# EUROPEAN PATENT APPLICATION

(11) **EP 1 974 737 A1**
(43) Date of publication of application: **01.10.2008**
(21) Application number: 07104791.4
(22) Date of filing: 23.03.2007
(51) Int. Cl.: A61K 36/185, A61K 36/24, A61K 36/28, A61K 36/42, A61K 36/47, A61K 36/48, A61K 36/51, A61K 36/59, A61K 36/9066, A61K 45/06, A61P 3/10

(54) **Composition for the treatment of diabetes mellitus and metabolic syndrome**

(71) Applicant: Phyto Health Pharma B.V., 2289 CC Rijswijk (NL)
(72) Inventor: The designation of the inventor has not yet been filed
(74) Representative: van Loon, C.J.J.

(57) **Abstract**

The invention relates to the field of pharmacology. More specifically, the invention relates to a composition for the treatment of diabetes mellitus and/or the metabolic syndrome. Even more specifically, the invention relates to a herbal composition for treating the same. In one of its embodiments, the invention provides a composition comprising:
a) an anti hyperglycaemic agent
b) an anti inflammatory agent
c) an anti hyperlipidemic agent
d) an anti oxidant agent
e) a gastro-intestinal agent.

In a preferred embodiment, at least one of said agents is derived from a plant.

## Description

The invention relates to the field of pharmacology. More specifically, the invention relates to a composition for the treatment of diabetes mellitus and/or the metabolic syndrome. Even more specifically, the invention relates to a herbal composition for treating the same.

Diabetes mellitus is a chronic metabolic disorder characterized by a high blood glucose concentration (hyperglycemia), which is due to insulin deficiency or insulin resistance. It can be classified into type 1, type 2, "other types" or gestational diabetes (Table 1; National Diabetes Data Group, Diabetes 28;1039, 1979).
Acute signs and symptoms of uncontrolled diabetes are directly and immediately due to hyperglycaemia: glucosuria (glucose loss through urinating), polyuria (increased volume of urine), thirst, weight loss, fatigue, infection proneness, visual blurring (Harvey, Johns, Mc Kusick, Owens, Ross, 1988).
Diabetes mellitus is the most common disease associated with carbohydrate metabolism and is a major cause of disability and hospitalization. An estimated 30 million people worldwide had diabetes in 1985. By 1995, this number had shot up to 135 million. The latest WHO estimate for the number of people with diabetes, world-wide, in 2000 is 177 million. This will increase to at least 300 million by 2025 (WHO, Fact Sheet N° 236, Revised September 2002).
Chronic hyperglycaemia during diabetes causes glycation of body proteins that in turn leads to secondary complication affecting eyes, kidneys, nerves and arteries (Sharma et al., 1993). These may be delayed or lessened, by maintaining blood glucose values close to normal.
Diabetic Retinopathy is the leading cause of new blindness in people under 65 years of age in the USA (National Diabetes Advisory Board, 1987). Diabetes is a well-recognized risk factor for cataract and remains the major cause of blindness worldwide, accounting for half of the world's blind population (Thylefors, 1990). Currently surgical extraction of the involved lens is the sole treatment of cataract although effective, preventing or delaying the development remains the preferred approach.
Diabetes accelerates large vessel atherosclerosis, causing an increased incidence of peripheral vascular, coronary artery, and cerebrovascular disease (Collwell JA, 1985). About 60% of people with type II diabetes die of atherosclerotic cardiovascular disease; about 25% die of stroke (National Diabetes Advisory Board, 1987). Coexisting risk factors for atherosclerosis- especially smoking, hypertension, and increased LDL cholesterol or decreased HDL cholesterol add greatly to the risk of atherosclerosis in diabetes.
The number of deaths attributed to diabetes was previously considerably underestimated. A plausible figure is likely to be around 4 million deaths per year related to the presence of the disorder. This is about 9% of the global total. Many of these diabetes related deaths are from cardiovascular complications. Most of them are premature deaths when the people concerned are economically contributing to society. This situation is increasingly outstretching the health-care resources devoted to diabetes (WHO, Fact Sheet N° 236, Revised September 2002).
Because of its chronic nature, the severity of its complications and the means required to control them, diabetes is a costly disease, not only for the affected individual and his/her family, but also for the health authorities. Studies in India estimate that, for a low-income Indian family with an adult with diabetes, as much as 25% of family income may be devoted to diabetes care. For families in the USA with a child who has diabetes, the corresponding figure is 10%. The total health care costs of a person with diabetes in the USA are between twice and three times those for people without the condition. It was calculated, for example, that the cost of treating diabetes in the USA in 1997 was US$ 44 billion. In WHO's Western Pacific region a recent analysis of health care expenditure has shown that: 16% of hospital expenditure was on people with diabetes. Recent cost estimates, denied by similar methods to that quoted above for the USA, include those for Brazil (US$ 3.9 billion), Argentina (US$ 0.8 billion) and Mexico (US$ 2.0 billion). Each of these is an annual figure and is rising as diabetes prevalence increases (WHO, Fact Sheet N° 236, Revised September 2002).
Overall, direct health care costs of diabetes range from 2.5% to 15% annual health care budgets, depending on local diabetes prevalence and the sophistication of the treatment available. For most countries, the largest single item of diabetes expenditure is hospital admissions for the treatment of long-term complications, such as heart disease and stroke, kidney failure and foot problems. As the number of people with diabetes grows worldwide, the disease takes an ever-increasing proportion of national health care budgets. Introduction of cost-effective treatment strategies to reverse this trend are utmost necessary (WHO, Fact Sheet N° 236, Revised September 2002).
Since the largest single item of diabetes expenditure is hospital admissions for the treatment of long-term complications and since a diabetes epidemic is underway with many death related to long-term complications, the need for added benefits of new anti diabetic agents in the field of prevention or delay of long-term complications, is desired. It is also apparent that due to the side effects of the currently used drugs, there is a need for a safe agent with minimal adverse effects, which can be taken for longer durations.
In recent years the popularity of complementary medicine has increased considerably. Surveys conducted in Australia and USA indicate that almost 48,5% and 34% respondents had used at least one form of unconventional therapy including herbal medicine (Eisenberg et al., 1993; McLennan et al., 1996). Even WHO (1980) has suggested the evaluation of the potential of plants as effective therapeutic agents.

### Current treatment of diabetes

People with type 2 diabetes, especially those in the early stages, can manage their blood sugar through diet, weight loss, and physical activity. If this does not provide effective control, however, oral hypoglycaemic therapy and/or insulin are/is prescribed by doctors. There are five classes of medications available. Each has a different working mechanism (Table 3) and has its own side effects (Table 2). If one medication is not sufficient to lower the blood glucose levels to a desired extent, doctors may combine two or three diabetes treatment medications.

### Sulphonylureas

Sulphonylureas, such as Glucotrol® and Micronase®, are commonly prescribed medications for diabetes treatment. Sulphonylureas work by helping the body make insulin. They can be used alone or with other medications.

### Biguanides

Biguanides, such as Metformin (Glucophage®), help the body use insulin more effectively. It is often used by people who are overweight, since it also helps with weight control. It can be taken alone or with other medications.

### Alpha-glucosidase inhibitors

Alpha-glucosidase inhibitors, such as Precose® and Glyset®, work by slowing down the absorption of sugar in the digestive tract. They are often used in combination with other diabetes treatment medications.

### Thiazolidinediones

The overall action of thiozolidinediones is to make cells more sensitive to insulin. Medications include Avandia® and Actos®. Rezulin® was the first thiazolidinedione, but it was recently withdrawn from the market after it was determined it causes liver toxicity.

### Insulin

If oral medications do not control the blood sugar levels satisfactorily, insulin may be used for diabetes treatment. A person with type 2 diabetes needs insulin injections if his or her pancreas has stopped producing insulin altogether.

Although insulin therapy affords a tight and effective glycemic control, certain drawbacks such as oral ineffectiveness, short shelf live, requirement of constant refrigeration, parenteral therapy, and fatal hypoglycaemia in the event of excess dosage limit its usage. Moreover, pharmacotherapy with sulphonylureas and biguanides is also associated with side affects like hypoglycemia, weight gain, gastro-intestinal disturbances such as nausea, vomiting, diarrhea and lactate acidosis (regular renal function monitoring is, for example, recommended for metformin) (Rang and Dale, 1991). Thiazolidinediones (e.g. rosiglitazone), which reduce peripheral insulin resistance, are associated with weight gain due to water retention. Regular liver function monitoring is strongly recommended and thiazolidinediones are only prescribed as add on therapy.
The common anti diabetic drugs mostly have a single well-defined mechanism of action. There is a specific receptor system with which the active compound enters into a bond, leading to a specific cascade of reactions on molecular level and which ultimately leads to a specific effect on the glucose metabolism.
An exception to this is metformin, a member of the biguanides group of anti diabetic drugs. Its hypoglycemic effect is attributed to different actions (but all related to glucose in one way or the other) in contrast to most other anti diabetic drugs (sulphonylureas, thiazolidinediones) that have a single mechanism of action (Table 3).

In addition to the afore mentioned widely prescribed common anti diabetic drugs, some new drugs have been developed which only recently got a market approval. These new drugs and their classes are described below.

### Incretin Mimetics

Exenatide (Byetta®) exhibits many of the same effects as the human incretin hormone GLP-1. It has been shown to bind to and activate the known human GLP-1 receptor in vitro. Like GLP-1, Exenatide helps regulate glucose homeostasis through effects on multiple organs, including the pancreas, stomach, and liver:
- Increase in acute beta-cell response:
   o enhancement of glucose-dependent insulin secretion from beta cells in the pancreas;
   o restoration of first phase insulin response.
- Decrease in beta cell work load:
   o suppression of glucagon secretion from alpha cells in the pancreas, which leads to the reduction of hepatic glucose output from the liver;
   o slowing of gastric emptying, which allows for timely absorption of nutritients;
   o reduction of food intake.
Common side effects of Exenatide are: Acid or sour stomach, belching, diarrhoea, dizziness, feeling jittery, headache, heartburn, indigestion, nausea, stomach discomfort and vomiting.

### DPP-4 Inhibitors

GLP-1 is a 36 amino acid incretin hormone secreted from intestinal L-cells after the ingestion of a meal. The GLP-1 hormone in circulation acts at multiple levels to control glucose homeostasis. The active GLP-1 peptide is rapidly degraded by the DPP-4 enzyme which is expressed ubiquitously as a transmembrane ecto-enzyme and also found in the circulation in its soluble form. The inhibition of the enzyme DPP-4, leads to a larger circulating pool of the active form of GLP-1 which then confers multiple anti diabetes benefits. DPP4 inhibitors thus prolong the half-life of incretins resulting in increased insulin secretion, decreased glucagon secretion, delayed gastric emptying, and reduced food intake.

Januvia® is an example of a DPP-4 inhibitor. The most common side effects seen in Januvia® clinical studies were upper respiratory tract infection, sore throat, and diarrhoea.

### Inhaled Insulin

Exubera® consists of blisters containing human insulin inhalation powder, which are administered using the Exubera® Inhaler. A common side effect is hypoglycaemia. In clinical trials up to two years duration, patients treated with Exubera® demonstrated a greater decline in pulmonary function, specifically the forced expiratory volume in one second (FEV1) and the carbon monoxide diffusing capacity (DLCO), than comparator-treated patients. Because of the effect of Exubera® on pulmonary function, all patients should have spirometry (FEV1) assessed prior to initiating therapy with Exubera®. Assessment of pulmonary function (e.g., spirometry) is recommended after the first 6 months of therapy, and annually thereafter, even in the absence of pulmonary symptoms. Exubera® is contraindicated in patients who smoke or who have discontinued smoking less than 6 months prior to starting Exubera® therapy and in patients with underlying lung disease such as asthma and COPD.

Reference is further made to professional literature in which, current medical therapy and the side-effects, morbidity, epidemiology of diabetes mellitus and analysis of health care expenditure is reviewed in more detail.

As described above, current treatments are primarily directed to control glucose related processes in a diabetic patient. The present application describes medicaments that not only control glucose related processes, but that preferably also control oxidative stress, systemic inflammation and/or lipid abnormalities. In the case of diabetes mellitus, hyperglycemic control is important in long-term complications risk management, but the present application describes that it is not enough to treat hyperglycemia alone in the prevention of long term complications. Adequate focus on and treatment of proven risk factors as well as potential risk factors (oxidative stress, inflammation -sensitive plasma proteins) for diabetes associated coronary artery disease and diabetes associated renal disease is necessary. Integral treatment of diabetes mellitus offers hope of intervening to delay or prevent lesion progression and complications.

The present invention provides a composition that has a multifactor or multimodal mechanism. Moreover,, the present invention provides a composition that in respect of long term complication not only treats hyperglycemia, but also for example oxidative stress, inflammation and/or hyperlipidemia
In a first embodiment, the invention provides a composition comprising:
a) an anti hyperglycaemic agent
b) an anti inflammatory agent
c) an anti hyperlipidemic agent
d) an anti oxidant agent
e) a gastro-intestinal agent.
   As described in the experimental part herein, a composition of the invention is capable of at least in part reducing the blood glucose levels, i.e. the composition according to the invention comprises an anti hyperglycaemic agent.

Furthermore the herein described compositions also comprise anti-inflammatory properties. In a study by R. Yegnanarayan et al⁸ various models of inflammation were employed for testing the anti-inflammatory activity of extracts of *Curcuma Longa.* Carrageenin induced edema was taken as a prototype of exudative phase of inflammation and the granuloma pouch and cotton pellet tests were the methods involving proliferative phase of inflammation. The extracts of *C. Longa* showed significant anti-inflammatory activity in both exudative and proliferative inflammation and this activity was comparable to some extent with that of sodium salicylate, hydrocortisone, oxyphenbutazone and indomethacin.
The composition according to the invention is further capable of decreasing levels of triglycerides, VLDL (Very Low Density Lipoprotein), LDL (Low Density Lipoprotein), HDL (High Density Lipoprotein), and TC (Total Cholesterol), i.e. the composition according to the invention comprises an anti hyperlipidemic agent. Preferably, the level of at least one of said lipid components is significantly decreased. In an even more preferred embodiment, the levels of at least two or three lipidic components are significantly decreased.
It is thought that high blood glucose levels can cause the production of free radicals. An anti oxidant agent is capable of at least in part neutralising a free radical and hence an anti oxidant agent is beneficial in the treatment of diabetes mellitus (preferably type II or type I or pregnancy diabetes) or metabolic syndrome
A composition according to the invention further comprises a gastro-intestinal agent, i.e. an agent that allows proper absorption of the medication. This agent tones the digestion and improves at least partly the absorption of nutrients, which is often impaired in diabetes.
The above described composition is preferably used for the treatment of diabetes mellitus or metabolic syndrome. Hence, the invention provides a composition for the treatment of diabetes mellitus or metabolic syndrome comprising: a) an anti hyperglycaemic agent, b) an anti inflammatory agent, c) an anti hyperlipidemic agent, d) an anti oxidant agent and e) a gastro-intestinal agent. Preferably, said diabetes mellitus is diabetes mellitus type II. However, other types of diabetes, such as diabetes type I or pregnancy diabetes, can also be treated by using the above described composition.
As will be discussed later on in more detail, a composition of the invention can be present in any form, for example as a powder, or as a solution or as a solution in which at least one of the agents is present as a aggregate or as a colloid.
A composition of the invention can comprise more than one anti hyperglycaemic agent, anti inflammatory agent, anti hyperlipidemic agent, anti oxidant agent or gastro-intestinal agent. In a preferred embodiment the invention provides a composition comprising at least one, two, three, four, five or even six anti hyperglycaemic agent(s), anti inflammatory agent(s), anti hyperlipidemic agent(s), anti oxidant agent(s) or gastro-intestinal agent(s) or any combination thereof.
The agents in the composition of the invention can be derived from a plant, but active agents of which the molecular structure is known can also be added as a synthetically agent. In a preferred embodiment, at least one agent of composition of the invention is derived from a plant, i.e. the invention provides a composition comprising:
a) an anti hyperglycaemic agent
b) an anti inflammatory agent
c) an anti hyperlipidemic agent
d) an anti oxidant agent
e) a gastro-intestinal agent, wherein at least one of said agents is derived from a plant. In yet another preferred embodiment, at least two, three or four (preferably, but not necessarily, different kinds of) agents are derived from a plant. In a preferred embodiment, all agents are derived from a plant.

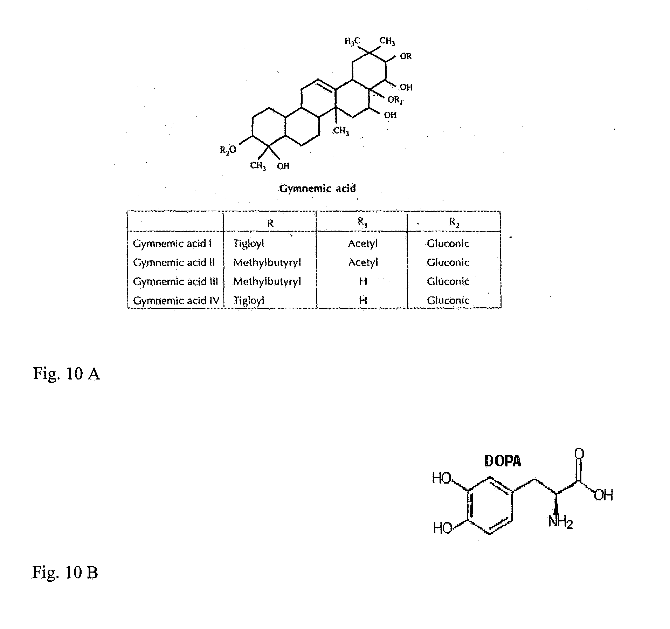

If an agent in the composition of the invention is derived from a plant, it can be present as a raw whole or crushed plant or as an extract (standardized extract, quantified extract, other extract, alcoholic/aqueous extract or any combination thereof). Since active compounds can be found in higher concentrations in specific parts of medicinal plants, but are present too a lesser extent in other parts of the medicinal plant as well, all parts of the medicinal plants (roots, fruits, seeds, leaves, wood, or whole plants) can be processed.
Most medicinal plants have advantageous secondary actions in addition to their primary pharmacological action and hence, one plant that is used to derive a certain activity from can at the same time provide one, two or three (or even more) secondary activities. Table 7 for example describes for 13 different plants which primary and secondary activities are known for said plants. It should be clear to the skilled person that Table 7 does not provide a limiting listing of the activities of the mentioned plants and/or plant extracts. Plants and/or plant extracts can equally well be divided based on their anti atherosclerotic or cardioprotective activity (in stead of anti hyperlipidemic), immunostimulant or immunomodulatory activity (in stead of anti inflammatory) or long evity promoting or cytoprotective activity (in stead of anti oxidant).
As described, active compounds from the medicinal plants with known molecular structures and pharmacological actions can be substituted by (synthetic) chemical compounds with similar molecular structures and pharmacological actions. Hence, in yet another preferred embodiment, the invention provides a composition comprising:
a) an anti hyperglycaemic agent
b) an anti inflammatory agent
c) an anti hyperlipidemic agent
d) an anti oxidant agent
e) a gastro-intestinal agent, wherein at least one of said agents is provided as a synthetic counterpart. Examples of such (synthetic) chemical compounds are curcumin (1.7-Bis (4-hydroxy-3-mehtoxyphenyl)- 1.6-hepatadiene-3.5-dione) or gymnemic acid (see Figure 10A) or L-dopa (see Figure 10B). Curcumin is for example used to replace Curcuma Longa or an extract thereof, gymnemic acid is for example used to replace Gymnema Sylvestre or an extract thereof and L-Dopa is for example used to replace Mucuna Pruriens or an extract thereof. Theoretically, all used extracts described herein can be replaced by a synthetic compound.
   However, in a preferred embodiment, the invention uses medicinal plants or plant extracts in the preparation of a composition according to the invention. In one of the preferred embodiments, the invention provides a composition comprising:

a) an anti hyperglycaemic agent
b) an anti inflammatory agent
c) an anti hyperlipidemic agent
d) an anti oxidant agent
e) a gastro-intestinal agent, wherein said at least one agent is derived from a plant and wherein said at least one of agent is an anti hyperglycaemic agent. Hence, at least one plant or plant extract is used that has as a primary activity an anti hyperglycaemic activity. In a preferred embodiment, the anti hyperglycemic agent is derived from one or more plants from the group of Gymnema Sylvestre, Momordica Charantia, Trigonella Foenum Graecum, Enicostemma Littorale, Mucuna Pruriens, Curcuma Longa, Holarrhena Antidysenterica, Emblica Officinalis, Swertia Chirata, Terminalia Belerica and Terminalia Chebula or a plant with comparable pharmacological action. Examples of plants with a comparable pharmacological action are for example provided in Table 8.

In yet another preferred embodiment, the invention provides a composition comprising:
a) an anti hyperglycaemic agent
b) an anti inflammatory agent
c) an anti hyperlipidemic agent
d) an anti oxidant agent
e) a gastro-intestinal agent, wherein at least one of said agents is derived from a plant, wherein said at least one agent is an anti inflammatory agent. Hence, at least one plant or plant extract is used that has as a primary activity an anti inflammatory activity. In a preferred embodiment, the anti inflammatory agent is derived from one or more plants from the group of Swertia Chirata, Curcuma Longa, Vernonia Anthelmintica, Enicostemma Littorale, Emblica Officinalis and Momordica Charantia or a plant with comparable pharmacological action. Examples of plants with a comparable pharmacological action are for example provided in Table 8.

In another preferred embodiment, the invention provides a composition comprising:
a) an anti hyperglycaemic agent
b) an anti inflammatory agent
c) an anti hyperlipidemic agent
d) an anti oxidant agent
e) a gastro-intestinal agent, wherein at least one of said agents is derived from a plant, wherein said at least one agent is an anti hyperlipidemic agent. Hence, at least one plant or plant extract is used that has as a primary activity an anti hyperlipidemic activity. In a preferred embodiment, the anti hyperlipidemic agent is derived from one or more plants from the group of Emblica Officinalis, Mucuna Pruriens, Trigonella Foenum Graecum, Terminalia Chebula, Terminalia Belerica, Curcuma Longa, Gymnema Sylvestre, Momordica Charantia and Swertia Chirata or a plant with comparable pharmacological action. Examples of plants with a comparable pharmacological action are for example provided in Table 8.

In yet another preferred embodiment, the invention provides a composition comprising:
a) an anti hyperglycaemic agent
b) an anti inflammatory agent
c) an anti hyperlipidemic agent
d) an anti oxidant agent
e) a gastro-intestinal agent, wherein at least one of said agents is derived from a plant, wherein said at least one agent is an anti oxidant agent. Hence, at least one plant or plant extract is used that has as a primary activity as an anti oxidant agent. In a preferred embodiment, the anti oxidant agent is derived from one or more plants from Curcuma Longa or Emblica Officinalis. In another preferred embodiment, said anti oxidant agent is only present as a secondary activity. In a preferred embodiment, the anti oxidant agent is derived from one or more plants from the group of, Mucuna Pruriens, Swertia Chirata, Terminalia Belerica and Terminalia Chebula or a plant with comparable pharmacological action. The amount of examples of plants with comparable pharmacological action (i.e. anti-oxidant) are vast and include, without being limiting, vegetables and fruits.

In a further preferred embodiment, the invention provides a composition comprising:
a) an anti hyperglycaemic agent
b) an anti inflammatory agent
c) an anti hyperlipidemic agent
d) an anti oxidant agent
e) a gastro-intestinal agent, wherein at least one of said agents is derived from a plant, wherein said at least one agent is an gastro-intestinal agent. Hence, at least one plant or plant extract is used that has as a primary activity a gastro-intestinal activity. In a preferred embodiment, the gastro-intestinal agent is derived from one or more plants from the group Jateorhiza Palmata, Holarrhena Antidysenterica and Curcuma Longa or a plant with comparable pharmacological action. Examples of plants with a comparable pharmacological action are for example provided in Table 8.

In yet another preferred embodiment, the invention provides a composition as described above, wherein the agents are present in the following amounts, based on total composition and preferably based on primary pharmacological action
a) 10 - 50 weight % of the anti hyperglycaemic agent
b) 10 - 30 weight % of the anti inflammatory agent
c) 10 - 50 weight % of the anti hyperlipidemic agent
d) 10 - 30weight % of the anti oxidant agent
e) 0.05 - 10 weight % of the gastro-intestinal agent
   In yet another preferred embodiment, the provided ranges reflect the total activity (i.e. primary as well as secondary) of the mentioned agents.
   In an even more preferred embodiment, the invention provides a composition as described above, wherein the agents are present in the following amounts, based on total composition and preferably based on primary pharmacological action

a) 20 - 40 weight % of the anti hyperglycaemic agent
b) 10 - 30 weight % of the anti inflammatory agent
c) 20 - 40 weight % of the anti hyperlipidemic agent
d) 10 - 30weight % of the anti oxidant agent
e) 0.05 - 10 weight % of the gastro-intestinal agent. In yet another preferred embodiment, the provided ranges reflect the total activity (i.e. primary as well as secondary) of the mentioned agents.

In a most preferred embodiment, the invention provides a composition as described above, wherein the agents are present in the following amounts, based on total composition and preferably based on primary pharmacological action
a) 30 weight % of the anti hyperglycaemic agent
b) 17 weight % of the anti inflammatory agent
c) 30 weight % of the anti hyperlipidemic agent
d) 18 weight % of the anti oxidant agent
e) 5 weight % of the gastro-intestinal agent. In yet another preferred embodiment, the provided ranges reflect the total activity (i.e. primary as well as secondary) of the mentioned agents. It is clear to the skilled person that minor deviations from the above mentioned percentages are within the scope of protection, for example a deviation of 10 % for each mentioned amounts for agents (a) to (d) and a deviation of 2 % for agent (e).

One of the preferred embodiments of the invention is a composition that is based on 13 plants and/or plant extracts. Preferably said composition comprises 6 plants and/or plant extracts that provide anti hyperglycaemic activity as a primary activity, 2 plants and/or plant extracts that provide anti-inflammatory activity as a primary activity, 3 plants and/or plant extracts that provide anti hyperlipidemic activity as a primary activity and 2 plants and/or plant extracts that provide a gastro intestinal improving effect as a primary activity. The invention therefore provides a composition comprising a part or an extract from Curcuma Longa, Enicostemma Littorale, Emblica Officinalis, Gymnema Sylvestre, Holarrhena Antidysenterica, Jateorhiza Palmata, Momordica Charantia, Mucuna Pruriens, Swertia Chirata, Terminalia Belerica, Terminalia Chebula, Trigonella Foenum Graecum and Vernonia Anthelmintica or a plant with comparable pharmacological action if compared to the mentioned plants. Examples of plants with a comparable pharmacological action are for example provided in Table 8. In a further preferred embodiment, the invention provides a composition comprising:
a) an anti hyperglycaemic agent
b) an anti inflammatory agent
c) an anti hyperlipidemic agent
d) an anti oxidant agent
e) a gastro-intestinal agent, comprising a part or an extract from Curcuma Longa, Enicostemma Littorale, Emblica Officinalis, Gymnema Sylvestre, Holarrhena Antidysenterica, Jateorhiza Palmata, Momordica Charantia, Mucuna Pruriens, Swertia Chirata, Terminalia Belerica, Terminalia Chebula, Trigonella Foenum Graecum and Vernonia Anthelmintica or a plant with comparable pharmacological action if compared to the mentioned plants. Examples of plants with a comparable pharmacological action are for example provided in Table 8.

In a preferred embodiment, the invention provides a composition comprising:
a) an anti hyperglycaemic agent
b) an anti inflammatory agent
c) an anti hyperlipidemic agent
d) an anti oxidant agent
e) a gastro-intestinal agent, comprising a part or an extract from Curcuma Longa, Enicostemma Littorale, Emblica Officinalis, Gymnema Sylvestre, Holarrhena Antidysenterica, Jateorhiza Palmata, Momordica Charantia, Mucuna Pruriens, Swertia Chirata, Terminalia Belerica, Terminalia Chebula, Trigonella Foenum Graecum and Vernonia Anthelmintica or a plant with comparable pharmacological action if compared to the mentioned plants, which comprises:
   a) 0.05 - 20 weight % of Curcuma Longa
   b) 0.05 - 20 weight % of Enicostemma Littorale
   c) 0.05 - 20 weight % of Emblica Officinalis
   d) 0.05 - 20 weight % of Gymnema Sylvestre
   e) 0.05 - 20 weight % of Holarrhena Antidysenterica
   f) 0.05 - 20 weight % of Jateorhiza Palmata
   g) 0.05 - 20 weight % of Momordica Charantia
   h) 0.05 - 20 weight % of Mucuna Pruriens
   i) 0.05 - 20 weight % of Swertia Chirata
   j) 0.05 - 20 weight % of Terminalia Belerica
   k) 0.05 - 20 weight % of Terminalia Chebula
   l) 0.05 - 20 weight % of Trigonella Foenum Graecum
   m) 0.05 - 20 weight % of Vernonia Anthelmintica
In a more preferred embodiment, all plants are present in a weight % range of 0.5-19 and even more preferably in a weight % range of 1-18 and most preferred in a weight % range of 2-16.
Each of the medicinal plants or extract components in each of the categories may be replaced by other medicinal plants with comparable (preferably comparable primary) pharmacological actions. To minimise the effect (if any) on the safety and efficacy profile, it is preferred to replace for example one, two, three or four of the mentioned medicinal plants with another plant with comparable (preferably comparable primary) pharmacological action. Examples of such replacement compositions are for example given in Table 9.
Besides replacement compositions, a composition according to the invention can be expanded by the addition of additional (plant or plant derived) components. However, medicinal plants or extract components with comparable pharmacological actions may also be withdrawn from each category. Examples are provided in Table 9.

A composition according to the invention is preferably used in the preparation of a pharmaceutical and hence in a preferred embodiment, the invention provides a pharmaceutical composition comprising:
a) an anti hyperglycaemic agent
b) an anti inflammatory agent
c) an anti hyperlipidemic agent
d) an anti oxidant agent
e) a gastro-intestinal agent or any of the specific embodiments describes above. For example a pharmaceutical composition comprising a part or an extract from Curcuma Longa, Enicostemma Littorale, Emblica Officinalis, Gymnema Sylvestre, Holarrhena Antidysenterica, Jateorhiza Palmata, Momordica Charantia, Mucuna Pruriens, Swertia Chirata, Terminalia Belerica, Terminalia Chebula, Trigonella Foenum Graecum and Vernonia Anthelmintica or a plant with comparable pharmacological action if compared to the mentioned plants. Examples of plants with a comparable pharmacological action are for example provided in Table 8. Examples of alternative compositions are provided in Table 9. A pharmaceutical of the invention can further comprise any pharmaceutical acceptable diluent, excipient, carrier or adjuvant.
   Such a pharmaceutical may be presented in any pharmaceutical acceptable form, for example a solution, a suspension, a powder, a granule, a tablet or a capsule. A pharmaceutical of the invention may be used as monotherapy or as a starting therapy which later on accompanied by other treatments or as an add on therapy, for example in combination with sulfonylurea, metformin or insulin. The described pharmaceutical is, for example, useful for patients who are on oral therapy, maximum tolerated therapy and insulin or insulin alone.

In yet another embodiment, the invention provides use of a composition comprising:
a) an anti hyperglycaemic agent
b) an anti inflammatory agent
c) an anti hyperlipidemic agent
d) an anti oxidant agent
e) a gastro-intestinal agent or any of the specific embodiments describes above, in the preparation of a medicament for the treatment of diabetes or metabolic syndrome. Treatment comprises curing of, alleviation of symptoms associated with or prophylaxis treatment of diabetes or metabolic syndrome. In a preferred embodiment, the invention provides the use of a part or an extract from Curcuma Longa, Enicostemma Littorale, Emblica Officinalis, Gymnema Sylvestre, Holarrhena Antidysenterica, Jateorhiza Palmata, Momordica Charantia, Mucuna Pruriens, Swertia Chirata, Terminalia Belerica, Terminalia Chebula, Trigonella Foenum Graecum and Vernonia Anthelmintica or a plant with comparable pharmacological action if compared to the mentioned plants in the preparation of a medicament for the treatment of diabetes or metabolic syndrome. Examples of plants with a comparable pharmacological action are for example provided in Table 8. Examples of alternative compositions are provided in Table 9.
   In a preferred embodiment, said diabetes is diabetes mellitus type II. Newly diagnosed patients (starting diabetes), uncomplicated diabetes as well as diabetes with complications can be treated. Moreover, prediabetes, i.e. a phase in which the glucose tolerance is impaired (impaired glucose tolerance (IGT) and/or impaired fasting glucose (IFG)) which is associated with an increased risk of diabetes, can be treated. In another preferred embodiment, said diabetes is diabetes mellitus type I or pregnancy diabetes.
   The metabolic syndrome is a combination of medical disorders that increase one's risk for cardiovascular disease. It affects a large number of people in a clustered fashion. In some studies, the prevalence in the USA is calculated as being up to 25% of the population. It is known under various other names, such as (metabolic) syndrome X and insulin resistance syndrome.

Treatment according to the invention is especially useful to delay or prevent the development of long-term complications associated with diabetes mellitus and the metabolic syndrome. The invention therefore also provide use of a composition as described herein in the preparation of a medicament, to delay or prevent the development of long-term complications associated with diabetes mellitus and the metabolic syndrome. As a result, the health care costs for diabetes patients are reduced.

The invention further provides a method for treating a person in need thereof comprising administering an effective of amount of a composition as described herein to said person. In a preferred embodiment, said person is suffering from or at risk of diabetes or metabolic syndrome.

The invention also provides a method for preparing any of the compositions described herein. Such a method for example comprises the steps of
- crushing a plant or a plant part or preparing an extract from a plant, wherein said plants are Curcuma Longa, Enicostemma Littorale, Emblica Officinalis, Gymnema Sylvestre, Holarrhena Antidysenterica, Jateorhiza Palmata, Momordica Charantia, Mucuna Pruriens, Swertia Chirata, Terminalia Belerica, Terminalia Chebula, Trigonella Foenum Graecum and Vernonia Anthelmintica or a plant with comparable pharmacological action if compared to the mentioned plants
- mixing the obtained crushed plant or plant part or extracts
- optionally drying the obtained mixture

Now that it is clear that an effective long term complications risk reducing medicament preferably comprises a multifold or more precisely a fourfold mechanism of action (see experimental part for explanation in respect of fourfold mechanism) the same result can also be obtained by combining conventional medicaments for the treatment of hyperglycaemia, inflammation, hyperlipidemia and free radicals.

The invention will be explained in more detail in the following, non-limiting examples.

### Experimental part

### 1.1 Drug Profile

### Internal Drug Identification Code:

ADM01

### Form and Presentation:

(1) biconvex tablet, red color coating, 500 mg or
(2) capsule shaped tablet, red color coating, 1062 mg

### Composition:

See Table 4 for amount and type of extract components per tablet of 500 mg. Content of Active Ingredients:
(1) 450 mg or (2) 675 mg
Excipients:
(1) Gum Acacia powder, Starch, Talc or (2) Microcrystalline cellulose, Ac-Di-Sol, Mg Stearate

### Indication:

Diabetes mellitus Type II and metabolic syndrome

### Dosage and Directions for Use (1):

Dose1: 3 dd 1 tabs, total 1,5 gram
Dose2: 2 dd 2 tabs, total 2 gram
Dose3: 2 dd 3 tabs, total 3 gram

Dosage is preferably increased every 4 weeks based upon the fasting plasma glucose levels.

It is clear to the skilled person that for example the size, color, the total amount of active ingredients or dosage scheme etc of a tablet can be varied by the skilled person. The herein described example is by no means limiting.

### Contra Indications:

No data is available for use during pregnancy. No data is available for use in case of renal and hepatic failure. No data is available for use by children.

### Safety:

ADM01 gives no habituation phenomena. If used as directed and within the ranges, ADM01 is a safe product. It does not cause hypoglycemia in single use, however, hypoglycemia can happen with concurrent use of concomitant anti hypoglycemic agents, such as sulfonylurea or insulin.

### 1.2 Efficacy

### In vitro studies

### Anti oxidant activity

In a 1,1-diphyenyl-picryl hydrazyl (DPPH) assay the antioxidant activity of ADM01 and some of its extract components were evaluated against Curcumin as a standard anti oxidant. The anti oxidant activity of ADM01 appeared to be more than that of standard used and the drug showed either additive or synergistic effect of all the extract components to have the antioxidant activity.

It is thought that high blood glucose levels can cause the production of free radicals. Therefore, antioxidants are likely to benefit in diabetes treatment.

### Insulinotropic activity

In an in vitro test using rat islets, the insulinotropic ability of ADM01 was investigated against standard (tolbutamide) and placebo control.

There was a significant raise (p< 0.05) in rate of insulin secretion in ADM01 treated rat islets in dose dependent manner at basal as well as stimulated glucose levels.
ADM01 showed significant (p< 0.05) insulinotropic activity when compared with control, at least comparable with that of the standard drug tolbutamide (Table 5).

### In vivo animal studies

### Normal rabbit

In normal rabbits the effect of ADM01 after a single dose oral administration on blood glucose levels was investigated. Results indicated a dose dependent reduction in blood glucose levels.

The maximum average percent blood glucose reduction with a dose of 140 mg/1.5 kg body weight was 26% and was produced at 3rd hour after ADM01 administration. The maximum average percent blood glucose reduction with a dose of 280 mg/1.5 kg body weight was 32,55% and was also produced at 3rd hour after ADM01 administration. The maximum average percent blood glucose reduction with a dose of 560 mg/1.5 kg body weight was 43,99% and was also produced at 3rd hour after ADM01 administration.

### Experimental diabetic rats

In another study the effect of ADM01 on blood glucose levels in experimental diabetic rats, using the Streptozotocin-Nicotinamide model, was investigated. After confirming the stabilization of diabetes mellitus in the experimental animals a single dose study was carried out in which the effect of a single dose administration of different dosages of ADM01 on blood glucose levels was investigated against vehicle control.
Results revealed a dose dependent significant reduction of blood glucose levels. A significant blood glucose reduction (p < 0.05) was observed with 500 mg/kg at 12^{th} hr compared to 0hr blood glucose levels (22.99 %). At a dose of 1000 mg/kg, blood glucose levels were significantly reduced (p < 0.05) at 8^{th} hr (15.45 %) and 12^{th} hr (40.46 %) when compared to 0hr blood glucose levels. After the single dose study, a 30-days study was carried out in which the effect of daily administration of ADMO1 on blood glucose levels was investigated. The effect of doses of 200, 400 and 800 mg/kg body weight respectively were investigated.
Serum blood glucose levels were significantly decreased (P < 0,05) and serum insulin levels significantly increased (P < 0.05) after 10, 20 and 30 days of treatment in all ADM01
treatment groups when compared to their base line (0 day) levels. Similarly blood glucose levels were significantly decreased (P < 0.05) and serum insulin levels significantly increased (P < 0.05) in tolbutamide treated diabetic animals.

### Human studies

### Open label study

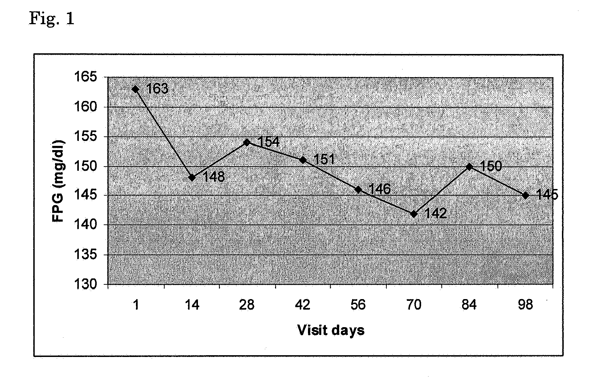

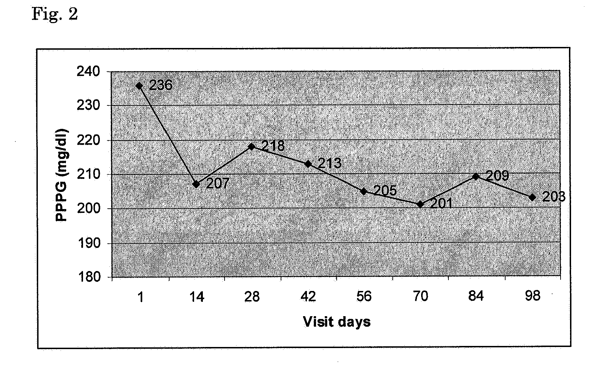

In an open label, non-comparator, multi centre study type 2 diabetes mellitus patients received ADM01 treatment for 84 days as add on therapy.
The interim analyses included 71 subjects who had completed till mid point of the study (day 42 of therapy). There was a significant reduction (p<0.05) in both fasting and post prandial plasma glucose levels. A significant effect (p < 0.05) was seen in reduction of serum LDL levels.
Preliminary end results indicate a significant improvement in levels of HbAlc (p= 0.025) of 0.2% after 84 days of treatment. Also Fasting Plasma glucose levels and Post Prandial Plasma Glucose levels show a significant decrease (p=0.001 resp. p<0.001). (see Figures 1 and 2)

### Double blind, placebo controlled, randomised study

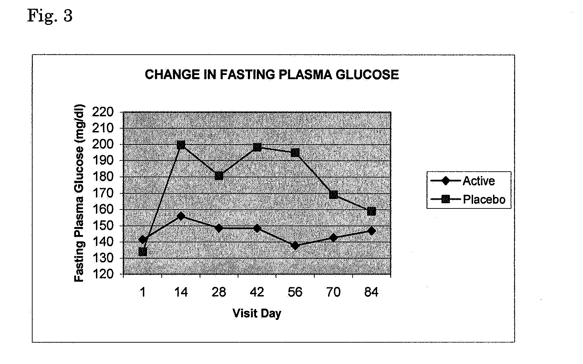

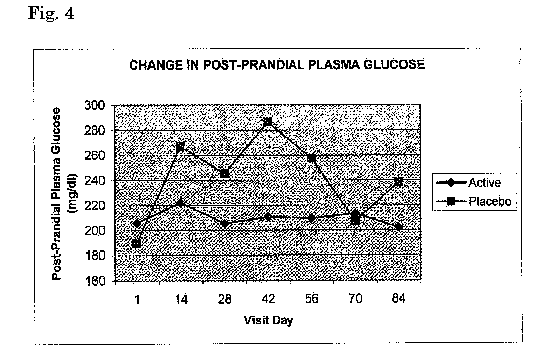

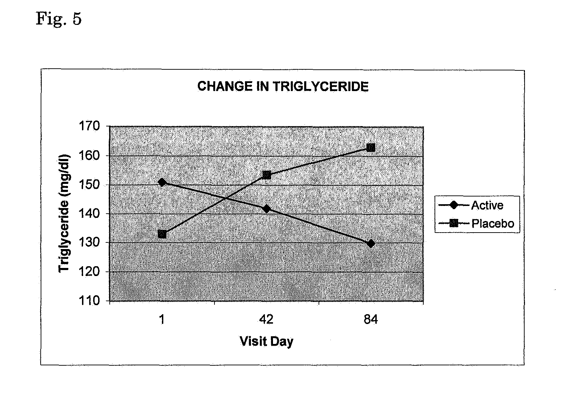

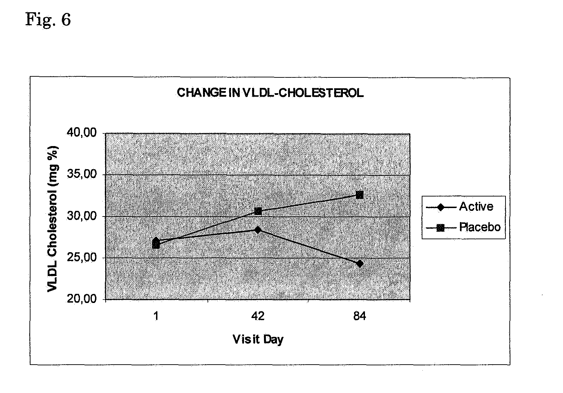

In a double blind, placebo controlled, randomized, parallel group, multi centre study type 2 diabetes mellitus patients received ADM01 or placebo treatment for 84 days. Conventional anti diabetic therapy was stopped and a wash out period of 5 days was allowed.
HbAlc significantly increased in both treatment groups, with no significant differences between the groups.
Fasting plasma glucose levels did not change statistically significant in the active group, but increased in the placebo group (p=0.001) (see Figure 3).
Post prandial plasma glucose levels did not change statistically significant in the active group (p=0.349), but increased in the placebo-group (p= 0.008) (see Figure 4).
Triglyceride and VLDL levels did not change in the placebo group, but decreased significantly in the active group. LDL, HDL and TC levels did not change significantly in the placebo group nor in the active group, however the levels showed decreasing trends in the active group (see Figures 5 and 6).

### 1.3 Safety

### Pre clinical tests

In toxicity tests in rat species up to 90 days no deaths occurred and no clinical signs were reported in any group. Body weights were unaffected by ADM01 treatment. Although there were some significant changes in both male and female rats at certain time intervals and at certain dose levels for parameters like feed intake, hematology and biochemical, the differences albeit significant, were very small and were within the normal range.
The NOAEL-No Observed Adverse Effect Level- was found to be at least 1000 mg/kg/day.

The mutagenecity of ADM01 was studied in a bacterial reverse mutase test using Salmonella Typhimurium. It was concluded that ADM01 up to 5.0 mg/plate, is non-mutagenic to all the five Salmonella Typhimurium tester strains viz., TA1537, TA1535, TA98, TA100 and TA102 when tested under the specified conditions.

The genotoxicity of ADM01 was investigated in an erythrocyte micronucleus assay. The results revealed that ADM01 did not produce any statistically significant increase (p> 0.05) in the micronucleus formation, while the positive control showed significant (p<0.001) increase in the presence of micronucleus when compared to the vehicle treated group.

In a study with normal rabbits gliclazide therapy was evaluated in the presence of single dose and repeated dose treatment with ADM01. There was no significant difference (p>0.05) between mean pharmacokinetic parameters of gliclazide with and without ADM01 co-administration, suggesting ADM01 not to alter the pharmacokinetics of sulfonylurea class of anti diabetic drugs.

### Clinical trials

Clinical studies with more that 100 patients who received ADM01 treatment varying from 6-12 weeks reveal no serious safety issues.

No significant changes from baseline levels in vital parameters (pulse rate, systolic and diastolic blood pressure, respiratory rate and temperature) (p > 0.05) nor in laboratory tests (Hb, WBC, ESR, Creatinine, SGPT) (p>0.05), except for RBC and micro albuminuria (p<0.05), nor in ECG parameters have been registered. No deaths or hypoglycaemia episodes have been reported.

### 1.4 Pharmacology

### 1.4.1 Mechanism of anti hyperglycaemic action

Pre clinical studies with extract components of ADM01 show their hypoglycemic effect to be attributed to different actions (Table 6):
1. stimulation of the release of endogenous insulin by the β-cells of the pancreas;
2. Improvement of the insulin-resistance;
3. Decrease in postprandial plasma glucose levels.

Therefore the ADM01 mechanism of anti hyperglycaemic action can be termed as a multifactor or multimodal_mechanism.

For some of the effects no specific receptor systems are identified and the mechanism is not completely understood (Table 6).

### 1.4.2 The metabolic syndrome

The metabolic syndrome is a combination of medical disorders that increase one's risk for cardiovascular disease. It affects a large number of people in a clustered fashion. In some studies, the prevalence in the USA is calculated as being up to 25% of the population. It is known under various other names, such as (metabolic) syndrome X and insulin resistance syndrome.

### Pathophysiology

The causes of metabolic syndrome are extremely complex and have only been partially elucidated. Most patients are older, obese and have a degree of insulin resistance. Insulin resistance is a generalized metabolic disorder, in which the body can't use insulin efficiently, leading to hyperinsulinemia and hyperglycemia A number of inflammatory markers including C-reactive protein and fibrinogen are often increased, indicating a state of systemic inflammation. Oxidative stress is often increased. Levels of triglycerides and low density lipoproteins may be increased and levels of high density lipoproteins may be decreased (lipid abnormalities).

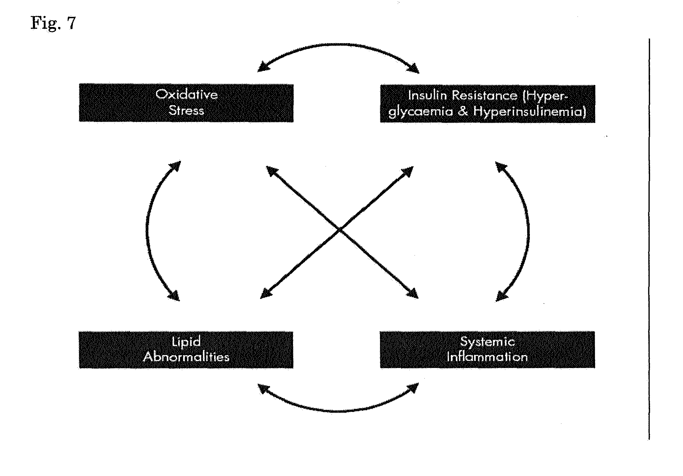

The metabolic syndrome thus is a combination of some essential medical disorders:
1. Insulin resistance (hyperinsulinemia and hyperglycemia);
2. Systemic Inflammation;
3. Oxidative stress and;
4. Lipid Abnormalities,
resulting in vascular damage (Figure 7). People with the metabolic syndrome are at increased risk of coronary heart disease and other diseases related to plaque build ups in artery walls (e.g., stroke and peripheral vascular disease) Abdominal obesity (excessive fat tissue in and around the abdomen) and elevated blood pressure are also often related with the metabolic syndrome.

### Treatment

### • Conventional treatment

The main treatment is lifestyle adjustments (i.e. caloric restriction and physical activity). However, drug treatment is often necessary. Generally, the individual diseases that comprise the metabolic syndrome are treated separately. Anti hypertensive agents may be prescribed for hypertension. Cholesterol lowering drugs may be used to lower LDL cholesterol and triglyceride levels, if they are elevated. Use of drugs that decrease insulin resistance e.g., metformin and thiazolidinediones may be prescribed.

### • ADM01: quadruple medication for the metabolic syndrome

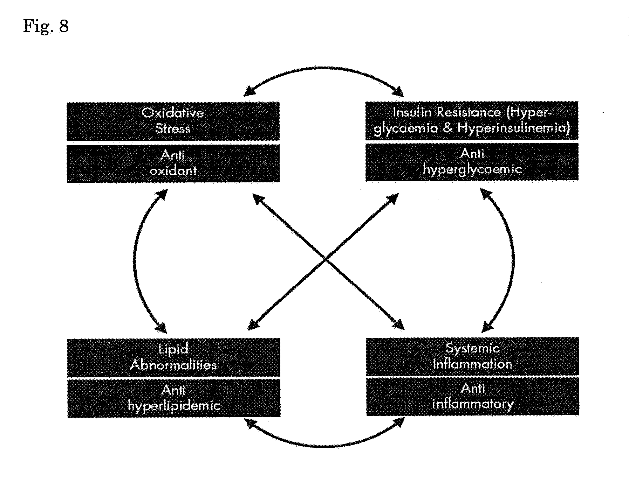

The essence of the concept behind ADM01 is its multifold, or more precisely its fourfold mechanism of action. Each of its extract components has either anti hyperglycaemic, anti inflammatory, anti oxidant or anti hyperlipidemic actions (Table 7). The fourfold mechanism of action makes the drug an efficient medication in the treatment of the metabolic syndrome and makes the separate treatment of the individual diseases that comprise the metabolic syndrome redundant (Figure 8). The extract component of Jateorhiza Palmata allows a proper absorption of the medication. The extract component tones the digestion and improves the absorption of nutrients.

### 1.4.3 Long term complications

ADM01 with its fourfold action of mechanism is likely to delay or prevent lesion progression and complications that are inherent to diabetes and the metabolic syndrome.

In the case of diabetes mellitus, hyperglycemic control is important in long term complications risk management, but it is not enough to treat hyperglycemia alone in the prevention of long term complications. Adequate focus on and treatment of proven risk factors as well as potential risk factors (oxidative stress, inflammation -sensitive plasma proteins) for diabetes associated coronary artery disease and diabetes associated renal disease is necessary. Integral treatment of diabetes mellitus offers hope of intervening to delay or prevent lesion progression and complications.

### 5.5 Extract component replacement study

In a replacement study, the extract components of ADM01 were replaced with extract components from medicinal plants with comparable pharmacologic actions and their efficacy was studied in male albino Wistar rats. The test animals were treated with Streptozotocin and Nicotinamide to induce type II diabetes.

The efficacy of in total 6 different test compounds with replaced extract components, indicated as ADM02 to ADM07, was compared with standard drug tolbutamide and diabetic control group. A full list of alternative medicinal plants that were used to replace the ADM01 extract components is provided in Table 8. Some examples of alternatives for ADM01 are provided in Table 9. Treatments were administered for 30 days.

Grouping was as follows.

| **Groups** | **Treatment** | **Dose** |
|---|---|---|
| Normal control | No treatment | Demineralised water |
| Diabetic control | No treatment | Demineralised water |
| Test Compound | ADM | Low dose |
| Test Compound | ADM | High Dose |
| Standard | Tolbutamide | 15mg/kg body weight |

The number of animals per group was 8 (n = 8).

### 1.6 Method of preparation

### Extraction

- Batches of medicinal plants are identified for authenticity by Quality Control (Q. C.) department.
- After receiving approval from the Q.C. department medicinal plant parts are sorted and crushed mechanically.
- The plant parts are then loaded in a stainless, vacuum-sealed extractor and an aqueous or alcoholic solution is added.
- The men strum is circulated for 4 hours and then removed by vacuum to a distillatory.
- The above process is repeated 4-5 times till the solution does not display any colour.
- The solution is distilled off and the sticky mass collected and dried to a powder.
- The extract is sent to Q.C. department.

### Dry Mixing

- After receiving approval from Q.C. department the standardized extracts of all extract components are weighed and mixed in prescribed quantities (ADM01 pre mix).
- ADM01 pre mix, Gum Acacia powder (10 %), starch (1 %) and talc (1 %) are weighed in required quantities depending upon the batch size and sieved through 80-mesh sieve.
- These ingredients are then loaded in a mass mixer and allowed to mix for 1 hour.

### Granulation

- After mixing for one hour, demineralised water is added and allowed to mix for 30 minutes.
- Wet granules are collected in steel trays.
- All trays are transferred in to tray drier.

### Drying

- Wet Granules are dried in tray drier at 80 °C for 8 hours each on two consecutive days till all granules are dried completely. The temperature is checked thrice at the interval of 2 hours during the drying process.
- Dried granules are then passed through multi mill at a slow speed.
- Dried granules are passed through 40-mesh sieve.
- Moisture content of the dried granules is checked.
- Total weight of the dried granules is checked to ensure that it is +/- 5 % which is within permissible limits.

### Lubrication

- Dried granules are transferred in to previously weighed mass mixture.
- Weighed quantity of lubricants are passed through 100-mesh sieve and mixed in mass mixture at high speed for 1 hour.
- Lubricated granules are transferred to cleaned weighed container having P.V.C bag inside the container.
- Granules are weighed & reconciled the yield for +/- 5 % deviation which is within permissible limits.
- Samples of lubricated granules are withdrawn & sent to Q. C. department.
- After receiving approval from the Q. C. department granules are taken for compression.

### Compression

- Machine Used: 16 Station Single Rotary Machines
- Punches: Size: 11.065 mm; Shape: Semi Convex
- The compression is done using compression machine of above specification.

### Coating

- Coating is done using Pathan Coater.
- Coating mixture included: ponceu 4, titanium dioxide, carmoisine black, M.D.C and iso propyl alcohol in coating pan with the help of spray gun.
Tablets are sent to the Q.C. department.

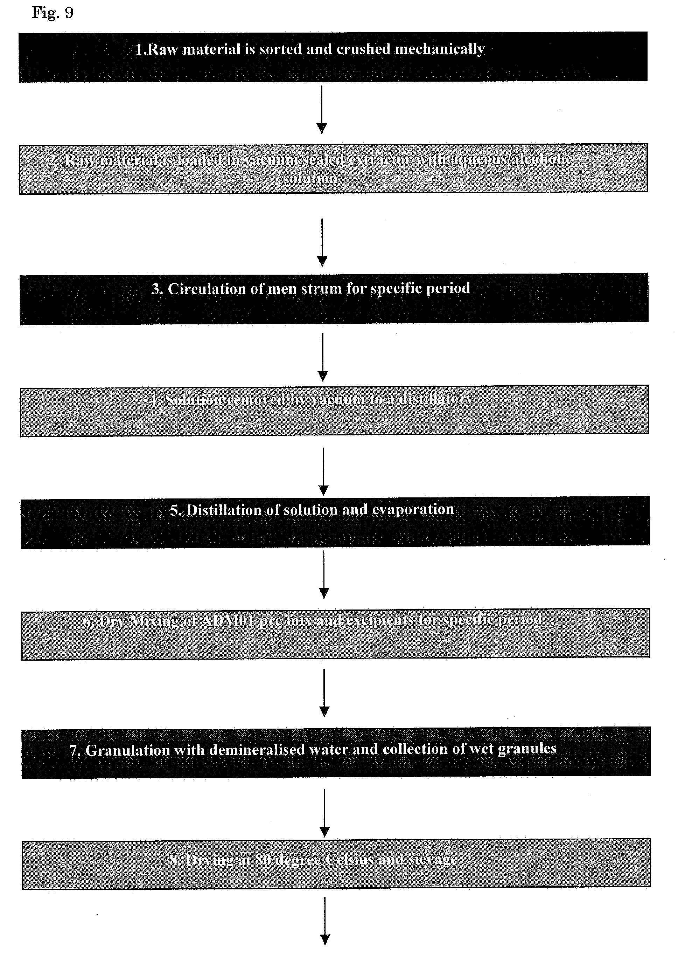

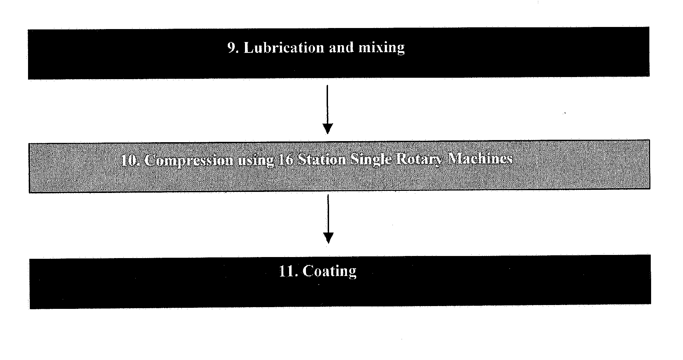

Figure 9 provides a chart that displays a method of preparation (process flow chart)

### 1.7 Additional experiments

Some of the additional experiments that will be performed are:
- A drug-drug interference study with for example metformine and other conventional anti-diabetic drugs;
- A genotoxicity study;
- Phase I safety study with healthy volunteers as well as with diabetic type II patients.

### 1.8 Concluding remarks

The invention is designed to treat not only diabetes type 2 but also the metabolic syndrome.

In the treatment of diabetes mellitus the ADM01 mechanism of anti hyperglycaemic action can be termed as a multifactor or multimodal mechanism (Table 6).
In the treatment of the metabolic syndrome the essence of the concept is its multifold, or more precisely its fourfold mechanism of action. Each of its extract components has either anti hyperglycaemic, anti inflammatory, anti oxidant or anti hyperlipidemic actions (Table 7). The fourfold mechanism of action makes the drug an efficient medication in the treatment of the metabolic syndrome and makes the separate treatment of the individual diseases that comprise the metabolic syndrome redundant (Figure 8).

A unique feature of ADM01 is its capacity to minimize the long-term complications of diabetes mellitus and the metabolic syndrome. The complications, which include macrovascular and microvascular diseases, are caused by systemic inflammation, increased oxidative stress, altered lipid levels, raised plasma glucose levels and can be precipitated by hypertension, smoking, low physical activity and other risk factors. ADM01 minimizes the long-term complications of diabetes mellitus and the metabolic syndrome by normalizing altered lipid levels- in particular it reduces serum cholesterol-, achieving an optimum glycemic control, influencing pathways that are part of inflammatory reactions and by reducing the oxidative stress.
ADM01 is a safe and well tolerated medicine.
ADM01 tones the digestion and improves the absorption of nutrients, which is often impaired in diabetes (gastropathy).

### Tables

**Table 1. Classification of diabetes mellitus**

| | |
|---|---|
| • Type 1: insulin-dependent diabetes mellitus (IDDM) | |
| • Type 2: non insulin-dependent diabetes mellitus (NIDDM) | |
| • Other types | |
| | - pancreatic disease |
| | - hormonal |
| | - drug or chemically induced |
| | - insulin receptor abnormalities |
| | - genetic syndromes |
| • Gestational diabetes | |

**Table 2: Side-effects**

| | |
|---|---|
| **Sulfonylurea** | |
| | hypoglycemia |
| | weight gain |
| | disturbance in liver function (rare) |
| | hypersensitivity reactions (rare) |
| | photosensitivity (rare) |
| | blood disorders (rare) |
| **Biguanides** | |
| | gastro-intestinal disturbances such as nausea, vomiting, diarrhoea |
| | lactate acidosis |
| | regular renal function monitoring is recommended |
| **Alpha-glucosidase inhibitors** | |
| | gastrointestinal problems including flatulence, diarrhea, and abdominal |
| | pain. |
| **Thiazolidinediones** | |
| | weight gain due to water retention |
| | regular liver function monitoring is strongly recommended |
| **Insulin** | |
| | Hypoglycaemia |
| | lipodystrophy |

as reported by H.P. Rang, M.M. Dale, 1991

**Table 3: Groups of anti diabetic drugs and their mechanisms of action**

| Group of Agents | Effect | Mechanism of Action |
|---|---|---|
| Sulfonylurea | insulin release from the beta cells is increased | Sulphonylureas bind to an ATP-dependant K⁺ channel on the cell membrane of pancreatic beta cells. This leads to an influx of potassium, a change in electric potential over the membrane, and opens voltage-gated Ca²⁺ channels. The rise in intracellular calcium leads to increased fusion of insulin granulae with the cell membrane, and therefore increased secretion of (pro) insulin. |
| | | |
| Thiazolidinediones | Insulin resistance is improved | Thiazolidinediones act by binding to PPARs (peroxisome proliferator-activated receptors), a group of receptors molecules inside the cell nucleus, specifically PPAR_{γ} (gamma). The normal ligands for these receptors are free fatty acids (FFAs) and eicosanoids. When activated, the receptor migrates to the DNA, activating transcription of a number of specific genes. By activating PPAR_{γ} insulin resistance is improved |
| | | |
| Alpha-glucosidase inhibitors | Postprandial glucose increase is reduced | Alpha-glucosidase inhibitors inhibit Apha-glucosidase in the intestinal wall which causes a delay in carbohydrate absorption. |
| | | |
| Biguanides | Insulin resistance is improved | The mechanism of Metformin's effect to reduce peripheral insulin resistance is not completely understood. |
| | | |
| | | The anti hyperglycemic action of Metformin has been attributed to a diminished intestinal absorption of carbohydrates, reduced gluconeogenesis, and increased peripheral glucose uptake. |

**Table 4: Amount and type of extract components per tablet of 500mg**

| # | Extract Component | Plant Part | Type of Extract | Quantity (mg) |
|---|---|---|---|---|
| 1. | Curcuma Longa | Root | Alcohol | 11.90 |
| 2. | Enicostemma Littorale | Whole | Alcohol | 39.64 |
| | | Plant | | |
| 3. | Emblica Officinalis | Fruit | Aqueous | 59.46 |
| 4. | Gymnema Sylvestre | Leaf | Alcohol | 23.78 |
| 5. | Holarrhena | Seed | Aqueous | 15.85 |
| | Antidysenterica | | | |
| 6. | Jateorhiza Palmate | Root | Alcohol/Aqueous | 29.76 |
| 7. | Momordica Charantia | Fruit | Alcohol | 47.56 |
| 8. | Mucuna Pruriens | Seed | Aqueous | 23.78 |
| 9. | Swertia Chirata | Whole | Alcohol | 19.82 |
| | | Plant | | |
| 10. | Terminalia Belerica | Fruit | Aqueous | 79.28 |
| 11. | Terminalia Chebula | Fruit | Aqueous | 59.46 |
| 12. | Trigonella Foenum | Seed | Aqueous | 15.85 |
| | Graecum | | | |
| 13. | Vernonia Anthelmintica | Seed | Alcohol | 23.78 |

**Table 5: Amount of insulin release by controls and ADM01, VL/051172/PHP**

| - insulinotropic ability of extracts are expressed as mean±SEM | | | |
|---|---|---|---|
| - Assay is repeated 3 times and average of 3 values are reported | | | |
| Name of the extract | Dose (in micrograms) | Amount of insulin release at basal glucose levels (2 mM) picomoles/L* | Amount of insulin during stimulated insulin release (glucose, 16 mM) picomoles/L* |
| Control (rat islets only) | No drug | 63.45±4.59 | 170.09±8.90 |
| | | | |
| Tolbutamide (standard Control) | 162(ImM) | 107.08±15.35 | 239.48±11.43 |
| | 10 | 93.34±6.52 | 181.57±10.27 |
| ADM01 | 100 | 236.93±15.29 | 324.28±14.19 |
| | 1000 | 510.39±19.91 | 670.26±43.69 |

**Table 6: Extract Components ADM01, Mechanism of Action**

| Effect | Mechanism of Action (Extract Component) |
|---|---|
| Stimulation of insulin-secreting beta cells | ADM01 stimulates beta cells functioning: |
| | -degranulation of beta cells (SWI) |
| | -increase of C-peptide level/enhanced endogenous insulin production (GS) |
| | -renewal/reeovery of b-cells (MC,GS) |
| | |
| Increase of glucose uptake and utilization in tissues | ADM01 increases peripheral glucose uptake and incorporation of glucose into glycogen and protein in the liver, kidney and muscle and reduces gluconeogenesis by the liver (MC, EL, MP). |
| | |
| | ADM01 increases the activities of the enzymes affording the utilisation of glucose by insulin dependent pathways: it controls phosphorylase levels, gluconeogenic enzymes and sorbitol dehydrogenase (GS). |
| | |
| | Increase in GLUT4 protein content in muscle plasma membrane (MC) |
| | |
| | Decrease in insulin resistance as measured by HOMA (TFG) |
| | |
| Postprandial glucose increase is reduced | ADM01 Delays carbohydrate absorption: |
| | Dietary fibre and delayed gastric emptying (TFG) Inhibition of glucose uptake incorporated with Na+ entry processes (GS) |

**Table 7: Primary and Secondary Actions**

| # | *Plant* | *Primary action** | *Secondary actions** |
|---|---|---|---|
| 1. | Curcuma Longa | II, V | I, III, IV |
| 2. | Enicostemma Littorale | I | II |
| 3. | Emblica Officinalis | III, V | I, II |
| 4. | Gymnema Sylvestre | I | III |
| 5. | Holarrhena Antidysenterica | IV | I |
| 6. | Jateorhiza Palmata | IV | |
| 7. | Momordica Charantia | I | II, III |
| 8. | Mucuna Pruriens | I | III, V |
| 9. | Swertia Chirata | I | II, III, V |
| 10. | Terminalia Belerica | III | I, V |
| 11. | Terminalia Chebula | III | I, V |
| 12. | Trigonella Foenum Graecum | I | III |
| 13. | Vernonia Anthelmintica | II | |

| | | | |
|---|---|---|---|
| * Notations *I = anti hyperglycemic effect* *II = anti inflammatory effect* *III = anti hyperlipidemic effect* *IV = gastro-intestinal improving effect* *V = anti oxidant effect* | | | |

**Table 8 List of medicinal plants with comparable pharmacologic actions**

| LIST OF ANTI-HYPERGLYCEMIC MEDICINAL PLANTS |
|---|
| **Latin names** |
| |
| Allium cepa |
| Allium sativum |
| Asparagus racemosa |
| Azadirachta indica |
| Pterocarpus marsupium |
| Tinospora cordifolia |
| Coccinia indica |
| Lagerstroemia speciosa |
| Ficus religiosa |
| Inula racemosa |
| Aegle marmelos |
| Ocimum sanctum |
| Eugenia jambolina |
| Phyllanthus niruri |
| Moringa oleifera |
| Ficus glomerata |
| Ficus benghalensis |
| Cinnamomum tamala |
| Salacia chinensis |
| Salacia reticulata |
| Centella asiatica |
| |

| |
|---|
| LIST OF ANTI-HYPERLIPIDEMIC MEDICINAL PLANTS |
| **Latin names** |
| |
| Allium satinum |
| Commiphora mukul |
| Terminalia arjuna |
| Acorus calamus |
| Heloptelea integrifolia |
| Rubia Cordifolia |
| Smilax china |
| Sphearanthus indicus |
| Colchicum luteum |

| LIST OF ANTI-INFLAMMATORY MEDICINAL PLANTS |
|---|
| **Latin names** |
| |
| Boswellia serrata |
| Withania somnifera |
| Ricinus communis |
| Pluchea lanceolata |
| Zingiber officinale |
| Glycyrrhiza glabra |
| Vitex negundo |
| Aloe vera |
| Tribulus terrestris |
| Ocimum sanctum |
| Crataeva nurvala |
| Acorus calamus |
| Commiphora mukul |
| Tinospora cordifolia |
| Rubia cordifolia |
| Cyperus rotundas |
| Moringa oleifera |
| Celastrus paniculatua |
| Aegle marmelos |
| Stereospermum suaveolens |
| Gmelina arborea |
| Oroxylum indicum |
| Premna mucronata |
| Solanum indicum |
| Solanum xanthocarpum |
| Desmodium gyrens |
| Uraria picta |
| |

| LIST OF GASTRO-INTESTINAL AGENTS |
|---|
| |
| **Latin names** |
| |
| Cyperus rotundus |
| Carica papaya |
| Zingiber officinale |
| Curcuma longa |
| Menth spicata |
| Anethum sowa |
| |
| Foeniculum vulgare |
| Plumbago zeylanica |
| Piper nigrum |
| Cuminum cyminum |
| Carum bulbocastom |
| Foerula asafetida |
| Punica granatum |
| Zingiber officinale |
| Garcinia indica |
| Oxalis corniculata |
| Aconitum heterophyllum |
| |

**Table 9 examples of ADM01 replacement composition**

| Latin Name | Quantity | Quantity |
|---|---|---|
| | | |
| **ADM01** | | |
| Gymnema Sylvestre | 5.29% | |
| Enicostemma Littorale | 8.81% | |
| Holarrhena Antidysentrica | 3.52% | |
| Mucuna Pruriens | 5.29% | |
| Emblica Officinalis | 13.21% | |
| Terminalia Chebula | 13.21% | |
| Terminalia Belerica | 17.62% | |
| Trigonella Foenum Graceum | 3.52% | |
| Momordica Charantia | 10.57% | |
| Swertia Chirata | 4.40% | |
| Vernonia Anthelmintica | 5.29% | |
| Jeteorrhiza Palmata | 6.62% | |
| Curcuma Longa | 2.65% | |
| | 100.00% | |
| | | |

| **ADM02** | | |
|---|---|---|
| Enicostemma Littorale | | 8.81% |
| Mucuna Pruriens | | 5.29% |
| Terminalia Chebula | | 13.21% |
| Terminalia Belerica | | 17.62% |
| Trigonella Foenum Graceum | | 3.52% |
| Vernonia Anthelmintica | | 5.29% |
| Eugenia Jambolana | | 5.29% |
| Coccinia Indica | | 10.57% |
| Tinospora Cordifolia | | 3.52% |
| Boswellia Serrata | | 4.40% |
| Ficus Racemosa | | 2.65% |
| Ocimum Sanctum | | 13.21% |
| Zingiber Officinale | | 6.62% |
| | | 100.00% |

| **ADM03** | | |
|---|---|---|
| Gymnema Sylvestre | 5.29% | |
| Momordica Charnatia | 10.57% | |
| Lagerostroemia Speciosa | 3.52% | |
| Pterocarpus Marsupium | 8.81% | |
| Allium Sativum | 13.21% | |
| Achryranthens Aspera | 17.62% | |
| Holorrhenna Antidysentrica | 3.52% | |
| Swertia Chirata | 4.40% | |
| Curcuma Longa | 2.65% | |
| Withania Somnifera | 5.29% | |
| Emblica Officinalis | 13.21% | |
| Phyllantus Niruri | 5.29% | |
| Jeteorhiza Palmata | 6.62% | |
| | 100.00% | |

| **ADM04** | | |
|---|---|---|
| Eugenia Jambolana | | 5.29% |
| Coccinica Indica | | 10.57% |
| Lagerostromia Speciosa | | 3.52% |
| Pterocarpus Marsupium | | 8.81% |
| Allium Sativum | | 13.2% |
| Achrantjus Aspera | | 17.62% |
| Tinospora Cordifolia | | 3.52% |
| Triphala | | 4.4% |
| Ficus Racemosa | | 2.65% |
| Withania Somnifera | | 5.29% |
| Ocimum Sanctum | | 13.21% |
| Phyllanthus Niruri | | 5.29% |
| Zingiber Officinale | | 6.62% |
| | | 100.00% |

| **ADM05** | | |
|---|---|---|
| G. Sylvestre | 5.29% | |
| E. Littorale | 8.81% | |
| M. Pruriens | 5.29% | |
| T. Foenumgraceum | 3.52% | |
| M. Charantia | 10.57% | |
| Swertia Chirata | 4.40% | |
| Total Actives | 37.88% | |
| Excipient (non-calorific) | 62.12% | |
| Total | 100% | |

| **ADM06** | | |
|---|---|---|
| G. Sylvestre | | 5.29% |
| E. Littorale | | 8.81% |
| M. Pruriens | | 5.29% |
| E. Officinalis | | 13.21% |
| T. Chebula | | 13.21% |
| T. Belerica | | 17.62% |
| T. Foenumgraceum | | 3.52% |
| M. Charantia | | 10.57% |
| Swertia Chirata | | 4.40% |
| Total Actives | | 81.92% |
| Excipient (non-calorific) | | 18.08% |
| Total | | 100% |

| **ADM07** | | |
|---|---|---|
| G. Sylvestre | 5.29% | |
| E. Littorale | 8.81% | |
| H. Antidysentrica | 3.52% | |
| M. Purensis | 5.29% | |
| E. Officinalis | 13.21% | |
| T. Chebula | 13.21% | |
| T. Belerica | 17.62% | |
| T. Foenumgraceum | 3.52% | |
| M. Charantia | 10.57% | |
| Swertia Chirata | 4.40% | |
| V. Anthelmintica | 5.29% | |
| C. Longa | 2.65% | |
| Total Actives | 93.38% | |
| Excipient (non-calorific) | 6.62% | |
| Total | 100% | |

### Description of Figures

### Figure 1

Change in fasting Plasma Glucose, VL/050419/PHP

### Figure 2

Change in Post Prandial Plasma Glucose, VL/050419/PHP

### Figure 3

Profiles, Fasting Plasma Glucose (mg/dl) (ITT), 3086/PHP

### Figure 4

Profiles, Post-Prandial Plasma Glucose (mg/dl) (ITT), 3086/PHP

### Figure 5

Profiles, Triglyceride (mg/dl) (PP), 3086/PHP

### Figure 6

Graph 6: Profiles, VLDL-Cholesterol (mg %) (PP), 3086/PHP

### Figure 7

The metabolic syndrome, a combination of some essential medical disorders

### Figure 8

ADM01; quadruple medication for the metabolic syndrome

### Figure 9

Method of preparation, process flow chart

### Figure 10

A) Gymnemic acid
B) 1 -3:4-dihydroxyphenylalanine (L-dopa)

### References

1. WHO, Fact sheet No. 236, Revised September 2002
2. Pharmacology, H.P. Rang, M.M. Dale, 1991
3. Drug information for the Health Care Professional, The United States Pharmacopoeial Convention, Inc., 1990
4. Martindale, The Extra Pharmacopoeia, James E.F. Reynolds, 1989
5. The Principles and Practice of Medicine, Harvey, Johns, Mc Kusick, Owens, Ross, 1988
6. Review of Medical Pharmacology, F.H. Meyers, Ernest Javetz, Alan Goldfien, 1980
7. The Pharmacological basis of Therapeutics, Louis S. Goodman, Alfred Gilman, 1965
8. R Yeganarayan, AP Saraf, JH Balwani, Comparison of anti-inflammatory activity of various extracts of Curcuma Longa. Indian J Med Res, 64 (1976) 601-608.

## Claims

1. A composition comprising:
a) an anti hyperglycaemic agent
b) an anti inflammatory agent
c) an anti hyperlipidemic agent
d) an anti oxidant agent
e) a gastro-intestinal agent.

2. A composition according to claim 1, wherein at least one of said agents is derived from a plant.

3. A composition according to claim 1, wherein at least one of said agents is a synthetic compound.

4. A composition according to claim 2, wherein said at least one agent is the anti hyperglycaemic agent.

5. A composition according to claim 4, wherein the anti hyperglycemic agent is derived from one or more plants from the group of Gymnema Sylvestre, Momordica Charantia, Trigonella Foenum Graecum, Enicostemma Littorale, Mucuna Pruriens, Curcuma Longa, Holarrhena Antidysenterica, Emblica Officinalis, Swertia Chirata, Terminalia Belerica and Terminalia Chebula or a plant with comparable pharmacological action.

6. A composition according to claim 2, wherein said at least one agent is the anti inflammatory agent.

7. A composition according to claim 6, wherein the anti inflammatory agent is derived from one or more plants from the group of Swertia Chirata, Curcuma Longa, Vernonia Anthelmintica, Enicostemma Littorale, Emblica Officinalis and Momordica Charantia or a plant with comparable pharmacological action.

8. A composition according to claim 2, wherein said at least one agent is the anti hyperlipidemic agent.

9. A composition according to claim 8, wherein the anti hyperlipidemic agent is derived from one or more plants from the group of Emblica Officinalis, Mucuna Pruriens, Trigonella Foenum Graecum, Terminalia Chebula, Terminalia Belerica, Curcuma Longa, Gymnema Sylvestre, Momordica Charantia and Swertia Chirata or a plant with comparable pharmacological action.

10. A composition according to claim 2, wherein said at least one agent is the anti oxidant agent.

11. A composition according to claim 10, wherein the anti oxidant agent is derived from one or more plants from the group of Curcuma Longa, Emblica Officinalis, Mucuna Pruriens, Swertia Chirata, Terminalia Belerica and Terminalia Chebula or a plant with comparable pharmacological action.

12. A composition according to claim 2, wherein said at least one agent is the gastro-intestinal agent.

13. A composition according to claim 12, wherein the gastro-intestinal agent is derived from one or more plants from the group Jateorhiza Palmata, Holarrhena Antidysenterica and Curcuma Longa or a plant with comparable pharmacological action.

14. A composition according to any one of claims 1 to 13, wherein the agents are present in the following amounts, based on total composition and based on primary pharmacological action
a) 10 - 50 weight % of the anti hyperglycaemic agent
b) 10 - 30 weight % of the anti inflammatory agent
c) 10 - 50 weight % of the anti hyperlipidemic agent
d) 10 - 30weight % of the anti oxidant agent
e) 0.05 - 10 weight % of the gastro-intestinal agent

15. A composition according to any one of claims 1, 2 and 4-14, comprising a part or an extract from Curcuma Longa, Enicostemma Littorale, Emblica Officinalis, Gymnema Sylvestre, Holarrhena Antidysenterica, Jateorhiza Palmata, Momordica Charantia, Mucuna Pruriens, Swertia Chirata, Terminalia Belerica, Terminalia Chebula, Trigonella Foenum Graecum and Vernonia Anthelmintica or a plant with comparable pharmacological action if compared to the mentioned plants.

16. A composition according to claim 15 which comprises:
a) 0.05 - 20 weight % of Curcuma Longa
b) 0.05 - 20 weight % of Enicostemma Littorale
c) 0.05 - 20 weight % of Emblica Officinalis
d) 0.05 - 20 weight % of Gymnema Sylvestre
e) 0.05 - 20 weight % of Holarrhena Antidysenterica
f) 0.05 - 20 weight % of Jateorhiza Palmata
g) 0.05 - 20 weight % of Momordica Charantia
h) 0.05 - 20 weight % of Mucuna Pruriens
i) 0.05 - 20 weight % of Swertia Chirata
j) 0.05 - 20 weight % of Terminalia Belerica
k) 0.05 - 20 weight % of Terminalia Chebula
l) 0.05 - 20 weight % of Trigonella Foenum Graecum
m) 0.05 - 20 weight % of Vernonia Anthelmintica

17. A pharmaceutical composition comprising a composition as claimed in any one of claims 1 to 16.

18. Use of a composition as claimed in any one of claims 1 to 16 in the preparation of a medicament for the treatment of diabetes or metabolic syndrome.

19. A method for treating a person in need thereof comprising administering an effective of amount of a composition according to any one of claims to said person.

20. A method according to claim 19, wherein said person is suffering from or at risk of diabetes or metabolic syndrome.
